# EUROPEAN PATENT APPLICATION

(11) **EP 4 682 106 A1**
(43) Date of publication of application: **21.01.2026**
(21) Application number: 24780237.4
(22) Date of filing: 26.03.2024
(51) Int. Cl.: C01G 9/02, A61K 8/27, A61Q 17/04

(54) **FATTY ACID-TREATED ZINC OXIDE PARTICLES, METHOD FOR PRODUCING SAME, AND DISPERSION AND COSMETIC HAVING SAME BLENDED THEREIN**

(30) Priority: 31.03.2023 JP 2023056907
(71) Applicant: Sakai Chemical Industry Co., Ltd., Sakai-shi, Osaka 590-8502 (JP)
(72) Inventor: ENDO, Rina, Iwaki-shi, Fukushima 971-8183 (JP); YOSHIDA, Ryohei, Iwaki-shi, Fukushima 971-8183 (JP); ASHIDA, Takuro, Sakai-shi, Osaka 590-0985 (JP)
(74) Representative: Isarpatent
(86) International application number: PCT/JP2024/011810
(87) International publication number: WO 2024/204123

(57) **Abstract**

Provided is a fatty acid-treated zinc oxide particle that has excellent dispersibility, does not exhibit excessive thickening even in a dispersion blended at a high concentration, and can provide a dispersion excellent in temporal stability. Disclosed is a fatty acid-treated zinc oxide particle characterized by having, in an FT-IR spectrum measured using a total reflection measurement method (ATR method), a ratio (I₂/I₁) of a maximum absorbance (I₂) at from 1500 to 1600 cm⁻¹ to a maximum absorbance (I₁) at from 1350 to 1500 cm⁻¹ of from 0.8 to 1.2, and a ratio (I₃/I₄) of a maximum absorbance (I₃) at from 1700 to 1720 cm⁻¹ to a maximum absorbance (I₄) at from 1750 to 1800 cm⁻¹ of 1.2 or less, wherein a primary particle size measured with a transmission electron microscope is 0.1 µm or less, and a volume-based mode diameter, according to a laser diffraction scattering method, of a dispersion liquid obtained by adding the powder to isopropyl alcohol and dispersed by ultrasonic irradiation using an ultrasonic homogenizer with a rated output of 600 W and a vibration amplitude of 100% at normal temperature for 1 minute is 0.30 µm or less.

## Description

### Technical Field

The present invention relates to fatty acid-treated zinc oxide particles, a method for producing the fatty acid-treated zinc oxide particles, and a dispersion and a cosmetic having the fatty acid-treated zinc oxide particles blended therein.

### Background Art

In the known art, titanium dioxide particles and zinc oxide particles have been used as an ultraviolet scattering agent in sunscreen cosmetics and the like because of their high ultraviolet shielding properties and transparency. When such a powder is untreated, because the powder has a hydrophilic surface, the powder may run away due to sweat or rain. Thus, especially for use in a cosmetic and the like, such a powder is often used after subjecting a particle surface to a water-repellent treatment.

As such a water-repellent treatment, a silicone treatment and an alkoxysilane treatment are generally used. However, silicone-treated products tend to be avoided in recent years, and zinc oxide subjected to a fatty acid treatment, which is a naturally occurring component, is required as an alternative product (for example, Patent Documents 1 and 2).

However, when the fatty acid treatment is performed, issues such as deterioration of performance due to formation of fatty acid soap including zinc ions and fatty acid eluted in water, thickening due to presence of unreacted free fatty acid, and precipitation of solid matter occur. For this reason, it is difficult to stably obtain a dispersion having a high concentration, and the performance is inferior to those obtained by a silicone treatment or an alkoxysilane treatment, so that fatty acid-treated zinc oxide is not widely distributed in the market.

### Citation List

### Patent Documents

Patent Document 1: JP 2016-196381 A
Patent Document 2: JP 2021-083334 A

### Summary of Invention

### Technical Problem

In view of the above, an object of the present invention is to provide a fatty acid-treated zinc oxide particle that has excellent dispersibility, does not exhibit excessive thickening even in a dispersion blended at a high concentration, and can provide a dispersion excellent in temporal stability.

### Solution to Problem

The present invention relates to a fatty acid-treated zinc oxide particle characterized by having, in an FT-IR spectrum measured using a total reflection measurement method (ATR method), a ratio (I₂/I₁) of a maximum absorbance (I₂) at from 1500 to 1600 cm⁻¹ to a maximum absorbance (I₁) at from 1350 to 1500 cm⁻¹ of from 0.8 to 1.2, and a ratio (I₃/I₄) of a maximum absorbance (I₃) at from 1700 to 1720 cm⁻¹ to a maximum absorbance (I₄) at from 1750 to 1800 cm⁻¹ of 1.2 or less, wherein a primary particle size measured with a transmission electron microscope is 0.1 µm or less, and a volume-based mode diameter, according to a laser diffraction scattering method, of a dispersion liquid obtained by adding the powder to isopropyl alcohol and dispersed by ultrasonic irradiation using an ultrasonic homogenizer with a rated output of 600 W and a vibration amplitude of 100% at normal temperature for 1 minute is 0.30 µm or less.

The aforementioned fatty acid is preferably at least one selected from the group consisting of stearic acid, isostearic acid, and palmitic acid.

The present invention provides a method for producing a fatty acid-treated zinc oxide particle, characterized by including:
Step (1) preparing a slurry by repulping zinc oxide particles as a primary product in a dispersion medium;
Step (2) neutralizing a fatty acid with a base to obtain a fatty acid salt aqueous solution; and
Step (3) performing a surface treatment by adding the obtained fatty acid salt aqueous solution to the zinc oxide slurry,
wherein the zinc oxide particles as the primary product are obtained by a production method including aging fine zinc oxide particles in water, in which a zinc salt is dissolved, and are subjected to Step (1) without drying, and Steps (2) and (3) are performed at a temperature equal to or higher than a melting point of the fatty acid.

In the zinc oxide particle as the primary product, a median diameter, according to a laser diffraction scattering method, of a dispersion liquid obtained by adding the powder to a 0.0025 mass% sodium hexametaphosphate aqueous solution and dispersed by ultrasonic irradiation using an ultrasonic homogenizer with a rated output of 600 W and a vibration amplitude of 100% at normal temperature for 3 minutes is preferably 0.30 µm or less.

An embodiment of the present invention is also a dispersion containing the fatty acid-treated zinc oxide particles.

An embodiment of the present invention is also a cosmetic containing the fatty acid-treated zinc oxide particles.

### Advantageous Effects of Invention

According to the present invention, it is possible to provide fatty acid-treated zinc oxide having a low environmental load and sufficient water repellency. By using the fatty acid-treated zinc oxide particles of the present invention, a dispersion and a cosmetic excellent in dispersibility and temporal stability can be obtained.

### Brief Description of Drawings

FIG. 1 is a diagram indicating FT-IR spectra of fatty acid-treated zinc oxide particles obtained in Example.
FIG. 2 is a diagram indicating FT-IR spectra of fatty acid-treated zinc oxide particles obtained in Comparative Example.
FIG. 3 is schematic view illustrating a measurement method of a primary particle size of the fatty acid-treated zinc oxide particles of the present invention.

### Description of Embodiments

Hereinafter, the present invention will be described in detail.

### Fatty Acid-treated Zinc Oxide Particle

The fatty acid-treated zinc oxide particles of the present invention suppress thickening of a dispersion even when blended at a high concentration, and can produce a dispersion excellent in temporal stability.

In order to obtain such an effect, it is assumed that the following three conditions need to be satisfied.
(1) An amount of fatty acid zinc (fatty acid soap) produced is suppressed.
(2) A residual amount of unreacted fatty acid is suppressed.
(3) The particles are easily dispersible.

Since various issues as described above are assumed to be caused by fatty acid zinc, unreacted fatty acid, and aggregated particles, the object of the present invention can be achieved by reducing these components.

When a treatment with a fatty acid in the present invention is performed, it is preferable to obtain treated particles in which a fatty acid is bound to the surface of zinc oxide particles. On the other hand, fatty acid zinc in a free state causes an undesirable effect. It is assumed that when zinc oxide reacts with water to generate zinc ions, the generated zinc ions reacts with the fatty acid to become fatty acid zinc in the free state. Thus, in the present invention, the step of surface treatment is adjusted so that such a reaction does not occur as much as possible.

Furthermore, if unreacted fatty acid remains, this also adversely affects the performance. Thus, it is also desired to prevent generation of excessive fatty acid.

It is assumed that when the particles have agglomeration, the agglomeration is loosened at the time of dispersion and an untreated surface is generated, so that thickening occurs when the particles are blended at a high concentration. Since easily dispersible particles are uniformly treated into particles without aggregation, the particles do not increase in viscosity even when mixed at a high concentration, and a low-viscosity dispersion can be obtained.

Since the fatty acid-treated zinc oxide particle of the present invention satisfies the above-described (1) and (2), IR peak intensity derived from the fatty acid zinc or the fatty acid is smaller than the IR peak intensity of the zinc oxide particle not subjected to a suitable surface treatment, and this is expressed by parameters (I₂/I₁) and (I₃/I₄) in the present invention.

The fatty acid-treated zinc oxide particle of the present invention is characterized by having, in an FT-IR spectrum measured using a total reflection measurement method (ATR method), a ratio (I₂/I₁) of a maximum absorbance (I₂) at from 1500 to 1600 cm⁻¹ to a maximum absorbance (I₁) at from 1350 to 1500 cm⁻¹ of from 0.8 to 1.2, and a ratio (I₃/I₄) of a maximum absorbance (I₃) at from 1700 to 1720 cm⁻¹ to a maximum absorbance (I₄) at from 1750 to 1800 cm⁻¹ of 1.2 or less.

In the above-described ratio (I₂/I₁), a value of the absorbance I₁ is the absorbance of the maximum peak among CH₃ symmetric bending vibration, COO⁻symmetric stretching vibration, and CH₃ asymmetric bending vibration detected at from 1350 to 1500 cm⁻¹, and a value of the absorbance I₂ is a value of the absorbance of the maximum peak of COO⁻ antisymmetric stretching vibration detected at from 1500 to 1600 cm⁻¹.

When the value of the ratio (I₂/I₁) is from 0.8 to 1.2, it means that the fatty acid is bound to a zinc oxide surface, and when the value is more than 1.2, it means that free fatty acid zinc is precipitated. When the ratio is less than 0.8, it means that the fatty acid is not sufficiently bound to the zinc oxide surface, and a large amount of unreacted fatty acid is present.

The lower limit of the ratio (I₂/I₁) is preferably 0.9, and the upper limit is preferably 1.1.

The fatty acid-treated zinc oxide particle of the present invention is further characterized in that the ratio (I₃/I₄) of the maximum absorbance (I₃) at from 1700 to 1720 cm^{- 1} to the maximum absorbance (I₄) at from 1750 to 1800 cm⁻¹ is 1.2 or less.

A value of the absorbance I₃ is the absorbance of the peak of C = O stretching vibration detected at from 1700 to 1720 cm⁻¹, and a value of the absorbance I₄ is a value of the absorbance of the peak of C = O stretching vibration of a four-membered ring, a five-membered ring, or an acid anhydride detected at from 1750 to 1800 cm⁻¹. In this production process, no substance corresponding to a four-membered ring, a five-membered ring, and an acid anhydride is present, and therefore no peak is detected. Thus, when the value of I₃/I₄ is more than 1.2, it means that unreacted fatty acid remains.

The ratio (I₃/I₄) is preferably 1.1 or less, and the lower limit thereof is not particularly limited, and is preferably 0.8 or more.

Further, since the fatty acid-treated zinc oxide particles of the present invention satisfy the above-described (3), a particle size distribution in weak dispersion is close to a primary particle size, which is expressed by a parameter called a mode diameter in the present invention.

The fatty acid-treated zinc oxide particle of the present invention is characterized in that a primary particle size measured based on a transmission electron micrograph is 0.1 µm or less, and a volume-based mode diameter, according to a laser diffraction scattering method, of a dispersion liquid obtained by adding the powder to isopropyl alcohol and dispersed by ultrasonic irradiation using an ultrasonic homogenizer with a rated output of 600 W and a vibration amplitude of 100% at normal temperature for 1 minute is 0.30 µm or less.

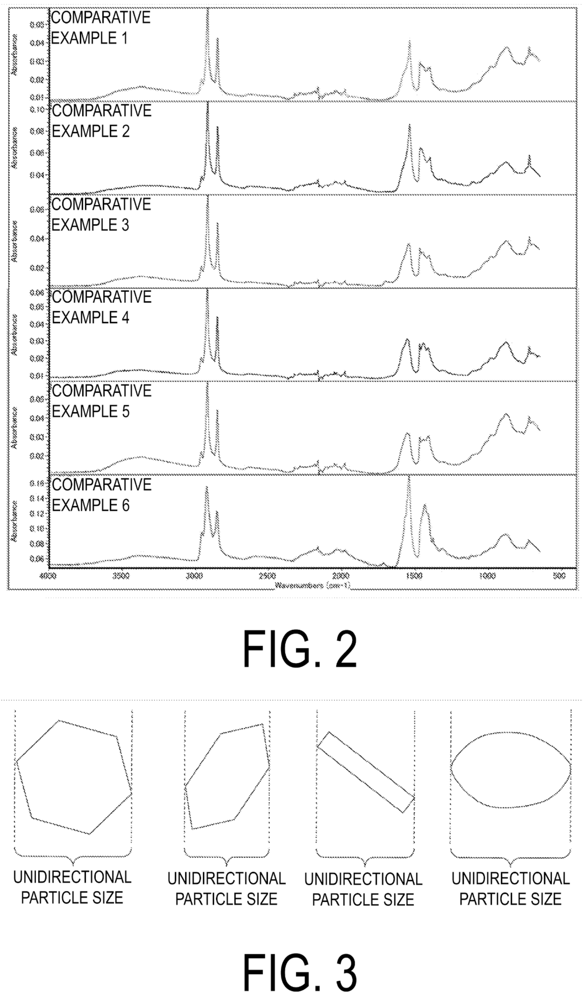

Specifically, the primary particle size in an embodiment of the present invention is a particle size (µm) defined as a unidirectional particle size (as illustrated in FIG. 3, distance between two parallel lines in one direction that are sandwiching a particle; for particles with all shapes in an image, measurement was performed for one direction) in a magnified field of view of 2000 to 50000 times an image taken by a transmission electron microscope JEM-1200EX II (available from JEOL, Ltd.), and determined by measuring unidirectional particle sizes for 250 primary particles in a TEM image, and calculating an average value of the cumulative distribution thereof. The primary particle size is more preferably 0.005 µm or more and less than 0.1 µm.

Furthermore, specifically, the mode diameter in the present invention is measured by the following method'
a slurry is prepared by adding 0.1 g of the fatty acid-treated zinc oxide particles to 50 ml of isopropyl alcohol and by ultrasonic irradiation using an ultrasonic homogenizer US-600E (available from Nippon Seiki Co., Ltd., rated output 600 W) with a vibration amplitude of 100% at normal temperature for 1 minute, and the volume-based mode diameter is measured by using a laser diffraction/scattering particle size distribution analyzer LA-960S (available from HORIBA, Ltd.). Furthermore, since the fatty acid-treated zinc oxide particles of the present invention have significantly high dispersibility, one of the characteristics thereof is that the particles are dispersed adequately by application of only a weak impact by ultrasonic wave. The mode diameter is more preferably 0.20 µm or less.

The fatty acid-treated zinc oxide particle of the present invention has a surface treatment layer with a fatty acid on the surface. As a water-repellent surface treatment agent, a fatty acid having high environmental adaptability is most desirable.

The surface treatment here is a water repellent treatment for reducing affinity of a surface of zinc oxide particles with water, and a surface treatment with a material that is easily dissolved in water after being subjected to the treatment or a material dispersed in water does not correspond to the "water repellent treatment" according to the present invention.

The fatty acid to be used in the present invention preferably has strong water repellency. Although a compound that forms chemical bond with an inorganic powder is preferable, even a compound that physically adsorbs the inorganic powder can obtain a certain degree of effect.

The fatty acid is preferred from the viewpoint that a higher fatty acid having from 10 to 30 carbon atoms can further improve the dispersibility of zinc oxide particles in the dispersion, and specifically, the fatty acid is preferably at least one selected from saturated fatty acids such as stearic acid, isostearic acid, myristic acid, lauric acid, and palmitic acid, and unsaturated fatty acids such as oleic acid.

Among them, stearic acid, palmitic acid, and isostearic acid are preferable from the viewpoint of being inexpensive, highly stable, and having strong water repellency particularly because of the simple structure.

In order to obtain a treatment agent having a smaller environmental load, it is preferable to use a plant-derived fatty acid.

The fatty acid-treated zinc oxide particle of the present invention may be one in which the particle surface of the zinc oxide particle as a primary product is coated with another inorganic compound. That is, the fatty acid-treated zinc oxide particle may be one in which the zinc oxide particles surface-treated with another inorganic compound may further be surface-treated with a fatty acid.

As a coating material, a known inorganic surface treatment material can be used, and for example, the surface is coated with one or more of oxides and hydroxides of Al, Si, Zr, Sn, etc.

Since zinc ions are eluted over time, the zinc oxide particles as a primary product have an issue that when the zinc oxide particles are blended in a cosmetic, an emulsion system may be broken, and other components are modified by surface catalytic activity of zinc oxide. However, when the zinc oxide particles are coated with an inorganic compound, outflow of zinc ions can be suppressed, and in such applications (for example, cosmetics and the like), the zinc oxide particles coated with the inorganic compound are preferable.

In particular, it is preferable to use one surface-treated with one or two of aluminum hydroxide and hydrous silicic acid from the viewpoint of being harmless to the environment and human body and having high activity suppression.

A coating amount of the inorganic surface treatment material is preferably from 1 to 30 mass% with respect to zinc oxide. Within this range, the activity is sufficiently suppressed, and the influence on characteristics of zinc oxide is small.

The lower limit is more preferably 3 mass%. The upper limit is more preferably 20 mass%.

The surface treatment method with the inorganic surface treatment material is not particularly limited, and can be performed by a well-known general method.

The fatty acid-treated zinc oxide particle of the present invention preferably has an apparent density of 0.5 g/ml or more. When the apparent density is 0.5 g/ml or more, the volume of the powder becomes smaller, which is preferred from the viewpoints of reducing amount of generated dust, facilitating charging into a packaging container, and enabling transporting a large amount of the powder during transport. Furthermore, it is preferred from the viewpoint of facilitating handling of the powder, for example, zinc oxide particles can be mixed in a container having a smaller volume when mixed with another component. The apparent density is more preferably 0.6 g/ml or more. A higher apparent density indicates a smaller volume of powder. The apparent density can be measured by a method described in JIS K 5101-12-1 "Test methods for pigments - Part 12: Apparent density or apparent specific volume - Section 1: Loose packing method".

The fatty acid-treated zinc oxide particle of the present invention preferably has a BET specific surface area of 25 m²/g or less. When the BET specific surface area is more than 25 m²/g, the particle size is smaller, and aggregation tends to occur. The BET specific surface area can be measured by the method described in Examples described below.

A carbon content in the fatty acid-treated zinc oxide particles of the present invention is preferably 0.5 mass% or more and less than 8.0 mass%. When the carbon content is less than 0.5 mass%, sufficient water-repellent effect may not be exhibited. When the carbon content is 8.0 mass% or more, a pure content of zinc oxide may be lowered and the performance may be deteriorated, and excessive addition may lead to precipitation of free fatty acid zinc. The carbon content is more preferably 1.0 mass% or more and less than 7.0 mass%. The carbon content can be measured by the method described in Example.

### Method for Producing Fatty Acid Zinc Oxide Particle

A method for producing the fatty acid-treated zinc oxide particles of the present invention is not particularly limited, and examples thereof include a method for producing fatty acid-treated zinc oxide particles, including: Step (1) of preparing a slurry by repulping zinc oxide particles as a primary product in a dispersion medium; Step (2) of neutralizing a fatty acid with a base to obtain a fatty acid salt aqueous solution; and Step (3) of perform a surface treatment by adding the obtained fatty acid salt aqueous solution to the zinc oxide slurry, wherein the zinc oxide particles as the primary product are obtained by a production method including a step of aging fine zinc oxide particles in water, in which a zinc salt is dissolved, and are subjected to Step (1) without undergoing a drying step, and Steps (2) and (3) are performed at a temperature equal to or higher than a melting point of the fatty acid.

Such a production method is also an embodiment of the present invention.

Step (1) is a step of preparing a slurry by repulping zinc oxide particles as a primary product in a dispersion medium. In the production method of the present invention, the preparation of the slurry of the zinc oxide particles in Step (1) is an important step. That is, Step (1) is characterized by the formation of fatty acid coating, not in a dried state in which the zinc oxide particles are aggregated, but in a dispersed slurry state.

Here, the zinc oxide particles used as raw materials in Step (1) are used for slurry preparation as is as the zinc oxide particles that underwent a synthesis reaction in an aqueous medium and that underwent no drying step. That is, preferably, no step of drying the particles is included between the production step of the particles to the step of fatty acid treatment. Consequently, aggregation of the particles is suppressed, and surface treatment is not performed for aggregated particles, and thus excellent dispersibility as described above can be achieved.

By this Step (1), each one of the zinc oxide particles is surface-treated, thus an excessive fatty acid coating amount is not required, and water repellency can be efficiently imparted.

In Step (1), for example, the slurry of the zinc oxide particles preferably contains, but not limited to, from 10 to 1000 g/L of zinc oxide particles.

The preparation method of the slurry is not particularly limited. For example, the preparation method may include filtering the zinc oxide particles obtained by the production step of the zinc oxide particles, then adding the zinc oxide particles to water without drying the zinc oxide particles, dispersing the zinc oxide particles at from 5 to 30°C for 10 to 30 minutes, and thus forming a uniform slurry containing a zinc oxide particle concentration of from 10 to 1000 g/L.

The dispersion medium is not particularly limited, and for example, an alcohol such as isopropanol and an organic solvent can be used; however, it is preferable to use water from the viewpoint of safety and production cost.

Step (2) is a step of neutralizing a fatty acid with a base to obtain a fatty acid salt aqueous solution, and is performed at a temperature equal to or higher than the melting point of the fatty acid. The concentration of the fatty acid salt aqueous solution is preferably 15 wt% or less, and more preferably 10 wt% or less from the viewpoint of production. Thus, the above-described temperature is more preferably a temperature at which solubility of the fatty acid salt in water is 10 wt% or more. For the above neutralization, it is preferable to use a commonly used base such as caustic soda (sodium hydroxide) and caustic potash (potassium hydroxide). Since the above melting point and solubility are determined by a composition of the fatty acid to be used, the temperature of Step (2) may vary. As an example, in a case where stearic acid is used as the fatty acid, the melting point of stearic acid is 70°C, and thus the step may be performed at 70°C or higher. When caustic soda is used as the base, the solubility of sodium stearate in water is 3 wt% at 70°C and 10 wt% at 75°C, and thus the temperature is more preferably 75°C or higher.

Step (2) is preferably performed under stirring. By stirring, the fatty acid and the fatty acid salt can be more sufficiently melted and dissolved.

The stirring means in Step (2) is not particularly limited, and examples thereof include stirring by an impeller, agitation by shaking, stirring by a mixer, and stirring by a stirrer.

Step (3) is a step of adding the obtained fatty acid salt aqueous solution to the zinc oxide slurry for surface treatment.

In the surface treatment step, stirring is preferably performed after the fatty acid salt aqueous solution is added to the slurry of the zinc oxide particles obtained in Step (1). By stirring, the fatty acid is easily uniformly adsorbed to the zinc oxide particles, and the surface treatment can be more efficiently performed.

The time for stirring is not particularly limited and is preferably 5 minutes or more. The stirring means is not particularly limited, and examples thereof include stirring by an impeller, agitation by shaking, stirring by a mixer, and stirring by a stirrer.

Step (3) of performing the surface treatment step is also performed at a temperature equal to or higher than the melting point of the fatty acid as in Step (2), and a temperature at which the solubility of the fatty acid salt in water is 10 wt% or more is more preferable. In Steps (2) and (3), the fatty acid is reacted with zinc oxide not in a solid state but in a liquid state, whereby a uniform surface treatment can be performed. When the temperature in Steps (2) and (3) is lower than the melting point of the fatty acid, the reaction of the fatty acid and the fatty acid salt with the zinc oxide may be insufficient. The term "the reaction is insufficient" herein includes a state in which the fatty acid and the fatty acid salt are present as a solid and are not uniformly chemically bonded or physically adsorbed to the zinc oxide. When the fatty acid and the fatty acid salt are not uniformly treated with the zinc oxide, even if the ratio (I₂/I₁) and the ratio (I₃/I₄) of the obtained fatty acid-treated zinc oxide particles are within the range of the present invention, issues such as agglomeration due to an untreated portion and difficulty in adsorption of a dispersant to a portion with an excessive amount of treatment may occur during preparation of the dispersion, and the viscosity may increase.

In Step (3), both the fatty acid salt aqueous solution and the zinc oxide slurry are preferably heated to a temperature equal to or higher than the melting point of the fatty acid.

Step (3) is a step of adding the fatty acid salt aqueous solution, and the addition is preferably performed in such a manner that an amount of the fatty acid added is 1.0 mass% or more with respect to the amount of the zinc oxide particles. When the addition amount is less than 1.0 mass%, the water repellency may be insufficient. The addition amount is more preferably 1.5 mass% or more. Furthermore, the addition amount is preferably 10 mass% or less. When the addition amount is more than 10 mass%, a simple fatty acid zinc that cannot be bound enough to the zinc oxide surface remains even by drying at a temperature equal to or higher than the melting points of the fatty acid and the fatty acid zinc in the drying step. This may cause the ratio (I₂/I₁) to fall outside the scope of the present invention. The addition amount is more preferably 8 mass% or less.

In this manner, after the surface treatment step is performed, the obtained slurry is aged for 0.5 hours to several ten hours while being stirred, and thus a fatty acid coating is formed on a surface of the zinc oxide particle.

In the aging, a small amount of another component may be added in the range that does not impair the effect of the present invention. For example, a dispersant or the like may be added.

The aging is preferably performed at from 45 to 110°C. In particular, the aging time may be from 0.5 to 24 hours. Since zinc oxide particles subjected to a uniform treatment can be obtained by conditions such as the aging temperature, aging time, and zinc oxide concentration, these conditions are preferably appropriately set according to the type and addition amount of fatty acid.

The fatty acid-treated zinc oxide particles thus obtained may be subjected to posttreatment such as pH adjustment, filtering, and water-washing as necessary.

In the pH adjustment step, the pH of the aged slurry is preferably adjusted to from 6 to 10, and more preferably from 7 to 8.

As a neutralizing agent, it is preferable to use a commonly used acid such as sulfuric acid or hydrochloric acid, and a zinc salt such as zinc sulfate or zinc chloride may be used.

The method for producing fatty acid-treated zinc oxide particles of the present invention preferably further includes a drying step. The drying is preferably performed at a temperature equal to or higher than the melting point of the fatty acid, and more preferably at a temperature equal to or higher than the melting point of the fatty acid zinc. By drying at a temperature equal to or higher than the melting points of the fatty acid and the fatty acid zinc, the remaining fatty acid and the excessively precipitated fatty acid zinc can be melted, and chemically bonded to the zinc oxide surface. As an example, when stearic acid is used as the fatty acid, the drying is preferably performed at 70°C or higher which is the melting point of stearic acid, and more preferably performed at 120°C or higher which is the melting point of zinc stearate.

The heating temperature in the drying step is not particularly limited, and it is preferable that there is no firing step of heating at 300°C or higher after the surface treatment. With such a configuration, it is possible to maintain the particle size and shape of the zinc oxide particles as the primary product, and it is possible to particularly suitably obtain the effect of the present invention as described above.

In addition, it is preferable to perform a pulverization step as necessary after the drying step. Examples of dry pulverization include an atomizer.

The zinc oxide particles as the primary product are not particularly limited; however, use of zinc oxide particles having a higher dispersibility is preferred because the dispersibility of the resulting fatty acid-treated zinc oxide particles is also increased. In particular, it is preferable to use zinc oxide particles having, according to a laser diffraction scattering method, a median diameter of 0.30 µm or less in a dispersion liquid dispersed in a 0.0025 mass% sodium hexametaphosphate aqueous solution by ultrasonic irradiation using an ultrasonic homogenizer with a rated output of 600 W and a vibration amplitude of 100% at normal temperature for 3 minutes.

As described above, by subjecting zinc oxide particles having excellent dispersibility to a fatty acid treatment, a uniform fatty acid coating layer is formed on each particle, and the obtained fatty acid-treated zinc oxide particles also have excellent easy dispersibility, and thus can be dispersed with a weak impact when blended in a cosmetic and the like. Meanwhile, when no impact is applied, aggregated particles can be formed by an appropriate cohesive force. Therefore, the handleability is improved.

The shape of the zinc oxide particle as the primary product is not particularly limited and is preferably a plate shape from the viewpoint of achieving excellent texture at the time of use of the resulting fatty acid-treated zinc oxide particles and excellent shielding performance against UV and the like. For example, zinc oxide particles having a primary particle size of less than 0.1 µm are preferred, zinc oxide particles having an aspect ratio of less than 2.5 are preferred, and zinc oxide particles having an oil absorption amount/BET specific surface area of 1.5 ml/100 m² or less are preferred. The oil absorption amount/BET specific surface area (ml/100 m²) is a value determined by dividing the value of the oil absorption amount (ml/100 g) by the value of the BET specific surface area (m²/g). A smaller value of the oil absorption amount/BET specific surface area indicates a lower oil absorption amount per unit area of the particle surface, less aggregation of the particles, and high independency and dispersibility of the particles. Examples of such primary product zinc oxide particles include zinc oxide particles obtained by a production method including a step of aging zinc oxide microparticulates having a primary particle size of 0.005 µm or more and 0.05 µm or less in water, in which zinc salt is dissolved. Specifically, for example, zinc oxide particles described in WO 2012/147888 are preferably used.

WO 2012/147888 describes a method for obtaining zinc oxide particles having excellent dispersibility by a production method including a step of aging zinc oxide microparticulates in water containing a zinc salt dissolved therein. After the zinc oxide particles are produced in the water by the method described above, the zinc oxide particles are preferably filtered and surface-treated by the method described above without being dried. As a result, the surface treatment can be performed for the zinc oxide particles, which are less aggregated, and the object of the present invention can be suitably achieved.

By the production method described in WO 2012/147888, zinc oxide particles having a primary particle size of less than 0.1 µm, an aspect ratio of less than 2.5, and an oil absorption amount/BET specific surface area of 1.5 ml/100 m² or less can be obtained. Such zinc oxide particles are particularly preferably used as the raw material.

For the fatty acid-treated zinc oxide particles of the present invention, another surface treatment may be further performed as necessary.

The surface treatment is not particularly limited, and examples thereof include a known treatment method such as an inorganic surface treatment for forming an inorganic oxide layer, such as a silica layer, an alumina layer, a zirconia layer, or a titania layer, or other various surface treatments. Furthermore, a plurality of types of surface treatments may be performed successively.

More specific examples of the surface treatment include a surface treatment by a surface treatment agent selected from the group consisting of an organoaluminum compound, an organotitanium compound, a polyhydric alcohol, and an alkanolamine. For such a surface treatment agent, the treatment amount can be appropriately set based on the particle size of the zinc oxide particle.

The fatty acid-treated zinc oxide particles of the present invention may be dispersed in water or an oil agent to form a dispersant.

Such a dispersion is also an aspect of the present invention.

### Dispersion and Cosmetic

The dispersion of the present invention preferably contains the fatty acid-treated zinc oxide particles of the present invention in a proportion of from 40 to 80 mass% in a total amount of the dispersion. The lower limit is more preferably 45 mass% and still more preferably 50 mass%.

Since the fatty acid-treated zinc oxide particles have the above-described effects, the dispersibility is good even in such a relatively high compounded amount range, the viscosity of the dispersion is low, and the viscosity is maintained over time.

The dispersion of the present invention preferably further contains polyhydroxystearic acid. The compound functions as a dispersant, and containing the compound is preferable in that the fatty acid-treated zinc oxide particles are well dispersed in a solvent.

When polyhydroxystearic acid is used, good dispersion can be achieved even in a small amount, and the polyhydroxystearic acid is particularly preferable in that the fatty acid-treated zinc oxide particles having a water-repellent surface treatment layer hardly flow with water even during use.

The hydroxyl group of hydroxystearic acid is preferably bonded to the carbon at position 12, and a polymerization degree of hydroxystearic acid is preferably from 3 to 12, and more preferably from 4 to 8. Examples of a commercially available product include Salacos HS-6C (available from The Nisshin OilliO Group, Ltd.) and ARLACEL P-100 (available from Uniqema of Gouda).

The compounded amount of the polyhydroxystearic acid is preferably from 0.1 to 10 mass% in the dispersion. The lower limit is more preferably 1 mass%, and the upper limit is still more preferably 7 mass% or less. The above proportion is particularly preferable in that the content of the solvent increases and the suitability for prescription at the time of blending the cosmetic is high.

The dispersion of the present invention preferably contains an oil agent as a medium for dispersing the fatty acid-treated zinc oxide particles. Although the oil agent is not particularly limited, an oil agent having no polysiloxane backbone is preferable from the viewpoint of environmental load, and a liquid oil that is liquid at normal temperature (from 15 to 25°C) is suitable from the viewpoint of dispersibility. Examples thereof include ester oil, fruit oil, and other oils and/or fats. Among them, ester oil and fruit oil are preferable in that they are easily used in cosmetics. Specific examples thereof include the following, and one kind or two or more kinds can be used.

Examples of the ester oil include ethylhexyl palmitate, isopropyl isostearate, ethyl oleate, octyldodecyl oleate, octyldodecyl myristate, diisostearyl malate, glyceryl tricaprylate, isooctyl isononanoate, isotridecyl isononanoate, isononyl isononanoate, ethylhexyl isononanoate, propylene glycol dicaprate, neopentylene glycol dicaprate, neopentylene glycol diethylhexanoate, cetyl ethylhexanoate, glyceryl tri-2-ethylhexanoate, jojoba oil, isopropyl myristate, isopropyl palmitate, isotridecyl isonanoate, polyglyceryl diisostearate, diglyceryl triisostearate, glyceryl tribehenate, dipentaerythrityl hexa(hydroxystearic acid/stearic acid/rosin acid), neopentyl glycol dioctoate, cholesteryl isostearate, cholesteryl hydroxystearate, cholesteryl stearate, lamolin fatty asid cholesteryl ester, phytosteryl oleate, N-lauroyl-L-glutamic acid di(cholesteryl/behenyld/octyldodecyl), and N-lauroyl-L-glutamic acid di(phytosteryl/2-octyldodecyl).

Examples of the fruit oil include olive fruit oil, litsea cubeba fruit oil, foeniculum vulgare (fennel) oil, carum carvi (caraway) fruit oil, piper nigrum (pepper) fruit oil, coriandrum sativum (coriander) fruit oil, juniperus communis fruit oil, vanilla planifolia fruit oil, citrus aurantium bergamia (bergamot) fruit oil, and citrus limon (lemon) fruit oil.

Examples of the other oils and/or fats include safflower oil, soybean oil, evening primrose oil, grape seed oil, rosehip oil, coconut oil, almond oil, sesame oil, wheat germ oil, corn oil, cottonseed oil, avocado oil, camellia oil, persic oil, castor oil, peanut oil, hazelnut oil, macadamia nut oil, meadowfoam oil, cacao fat, shea fat, wood wax, coconut oil, palm oil, palm kernel oil, beef tallow, horse fat, mink oil, milk fat, egg yolk oil, turtle oil, Japan wax, and camelia oil.

The compounded amount of the oil agent is preferably from 5 to 60 mass% in the dispersion. When the proportion is within the above-described range, the proportion is suitable from the viewpoint of being easily compatible with oil at the time of blending into a cosmetic and the like.

The lower limit is more preferably 10 mass%, and the upper limit is still more preferably 55 mass%.

In the dispersion of the present invention, in addition to the above-described components, a preservative, a pH adjusting agent, pure water, and the like may be appropriately blended according to the purpose.

In the dispersion of the present invention, an initial viscosity measured by the following evaluation method is preferably 1500 mPa·s or less, and more preferably 1000 mPa·s or less. Within this range, the viscosity is low, and bead separation after dispersion is easy.

### Initial Viscosity Evaluation Method:

Into a 9 ml screw bottle, 8 ml of the dispersion immediately after production was charged. Using a rotor No. 4 with a B-type viscometer (TVB-10 available from TOKYO KEIKI), the dispersion was rotated at 12 rpm, and the viscosity (25°C) after 60 seconds from the start of rotation was measured.

In the dispersion of the present invention, a temporal viscosity after 7 days as measured by the following evaluation method is preferably 2000 mPa·s or less, and more preferably 1800 mPa·s or less. Within this range, the viscosity is not high, the dispersion is easily taken out from a container or the like, and thus the handling is good.

### Temporal Viscosity Evaluation Method:

Into a 9 ml screw bottle, 8 ml of the dispersion immediately after production was charged and stored in a thermostatic bath at 40°C for 7 days. Using a rotor No. 4 with a B-type viscometer (TVB-10 available from TOKYO KEIKI), the dispersion was rotated at 12 rpm, and the viscosity (25°C) after 60 seconds from the start of rotation was measured.

A method for producing the dispersion of the present invention is not particularly limited.

Specifically, for example, the above-described fatty acid-treated zinc oxide particles, dispersant, and solvent are used as raw materials, and the resultant is mixed and stirred with other components, thereby obtaining the dispersion.

The dispersion of the present invention is preferably an oily dispersion substantially containing no water. In addition, the content of the components other than the fatty acid-treated zinc oxide particles, the dispersant, and the solvent described above is preferably 1 mass% or less, and more preferably 0.5 mass% or less. The dispersion may composed only of the above-described fatty acid-treated zinc oxide particles, dispersant, and solvent. The dispersion of the present invention can be used particularly as a raw material for cosmetic production. For this reason, it is preferable that components other than those described above are contained as little as possible, in order not to cause limitations on the prescription.

The dispersion of the present invention may contain inorganic particles other than zinc oxide. The inorganic particles that can be used in combination are not particularly limited, and examples thereof include titanium dioxide. In particular, when titanium dioxide is used in combination with zinc oxide, the issue of thickening tends to be remarkable, and thus the effect of the present invention is more remarkable when titanium dioxide is used in combination. When zinc oxide is used as an ultraviolet shielding agent in an ultraviolet protection cosmetic, titanium dioxide particles having different ultraviolet shielding areas may be used in combination, and thus the present invention is also useful in such a case.

In the present invention, the titanium dioxide particles used in combination are not particularly limited, and it is preferable to use titanium dioxide particles surface-treated with a fatty acid.

The coating amount of the fatty acid on the titanium dioxide particles is preferably from 4.0 to 8.5 mass% with respect to the titanium dioxide particles. Examples of the fatty acid include fatty acids similar to those used for zinc oxide.

In addition, similarly to the zinc oxide particles, particles treated with aluminum hydroxide and hydrous silicic acid and then treated with fatty acid are preferable.

The surface treatment of the titanium dioxide particles can be performed by a typical and known method or a method similar to the treatment of the zinc oxide particles described above.

The titanium dioxide particles preferably have an average primary particle size of 200 nm or less. When the average primary particle size of the titanium dioxide particles is more than 200 nm, a hiding power is large, the color becomes white, and transparency is low. The lower limit of the average primary particle size of the titanium dioxide particles is not particularly limited, and is usually 5 nm.

The average primary particle size of the titanium dioxide particles is calculated as an average value of particle sizes obtained by measuring particle sizes of 200 particles randomly selected under an electron microscope.

The shape of the titanium dioxide particles is not particularly limited, and any shape such as a spherical shape, a rod shape, a needle shape, a spindle shape, and a plate shape can be used. The average primary particle size in the case of a shape other than a spherical shape is defined by an average of lengths on a short axis side in the case of rod-shaped, needleshaped, and spindle-shaped particles, and is defined by an average of diagonal lengths of a surface in the case of a plate-like shape.

The major axis diameter/minor axis diameter (aspect ratio) of the titanium dioxide particles is preferably 9 or less. The aspect ratio of the titanium dioxide particles is calculated as an average value of the major axis diameter/minor axis diameter of 200 particles randomly selected under an electron microscope.

When inorganic particles such as titanium dioxide other than zinc oxide are used, a total amount of the fatty acid-treated zinc oxide particles and the inorganic particles such as titanium dioxide other than zinc oxide is preferably from 40 to 70 mass% with respect to the total amount of the dispersion.

In the case of using the inorganic particles other than zinc oxide, such as titanium dioxide, the inorganic particles may be blended within a range not hindering the object of the present invention, and for example, the compounded amount of the inorganic particles such as titanium dioxide other than zinc oxide is preferably twice or less the mass of zinc oxide.

The dispersion of the present invention is preferably a non-silicone-based dispersion. The term "non-silicone-based" refers to a material that does not substantially contain a silicone-based material, and the material preferably does not substantially contain inorganic particles surface-treated with a silicone-based oil agent, a silicone-based surfactant, or a silicone-based surface treatment agent. The silicone-based material is preferably 1 mass% or less, and more preferably 0.5 mass% or less in the total amount of the dispersion.

The dispersion of the present invention is suitably used for cosmetics. The cosmetic is not particularly limited, and the dispersion can be blended into cosmetics for external use on the skin or hair such as skin care products, hair products, makeup products, or UV blocking products. The form of the product is also not particularly limited, and the product can be applied in the form of an emulsion, a cream, a solid, a paste, a gel, a multilayer product, a mousse, or a spray.

In the cosmetic of the present invention, any aqueous component or oily component that can be used in the field of cosmetics can be used in combination. The aqueous component and the oily component are not particularly limited, and examples thereof include oils, surfactants, moisturizers, higher alcohols, sequestrants, natural water-soluble polymers, semisynthetic water-soluble polymers, synthetic water-soluble polymers, water-soluble and oil-soluble polymers, organic ultraviolet shielding agents, and various kinds of extracts.

In addition, if necessary, inorganic and organic pigments as various powders, various powders such as inorganic and organic clay minerals, inorganic and organic pigments that are metallic soap-treated or silicone-treated, coloring materials such as organic dyes, and components such as preservatives, antioxidants, dyes, thickeners, emulsion thickeners, pH adjusters, perfumes, cooling-sensation agents, antiperspirants, disinfectants, skin activators, anti-inflammatory agents, whitening agents, and film forming agent as other chemical components may be contained. Specifically, a desired cosmetic can be produced in the usual manner using any one or two or more types of the ingredients listed below. The compounded amounts of these ingredients are not particularly limited as long as the compounded amounts are in the range that do not impair the effect of the present invention.

In addition, an inorganic powder of an ultraviolet scattering agent such as titanium dioxide and cerium oxide may be used as long as the object of the present invention is not hindered.

The cosmetic of the present invention may be a non-silicone-based formulation, or may be a formulation containing a silicone-based material such as a silicone-based oil agent such as methyl silicone and methyl phenyl silicone depending on the purpose.

The oil is not particularly limited. Examples thereof include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, arachis oil, tea seed oil, kaya oil, rice bran oil, Chinese tung oil, Japanese tung oil, jojoba oil, germ oil, triglycerol, glycerol trioctanoate, glycerol triisopalmitate, cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol ester, POE hydrogenated lanolin alcohol ether, liquid paraffin, ozokerite, pristane, paraffin, ceresin, squalene, vaseline, microcrystalline wax, propylene glycol dicaprate, ethylhexyl palmitate, and isotridecyl isononanoate.

The surfactant described above is not particularly limited. Examples thereof include lipophilic nonionic surfactants, hydrophilic nonionic surfactants, and other surfactants.

The lipophilic nonionic surfactant described above is not particularly limited. Examples thereof include sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerin polyglycerin fatty acid esters, such as glycerol mono-cottonseed oil fatty acid, glycerol monoerucate, glycerol sesquioleate, glycerol monostearate, α,α'-glycerol oleate pyroglutamate, and glycerol monostearate malate; propylene glycol fatty acid esters, such as propylene glycol monostearate; hydrogenated castor oil derivatives; and glycerol alkyl ethers.

The hydrophilic nonionic surfactant described above is not particularly limited. Examples thereof include POE sorbitan fatty acid esters (for example, polysorbate 60), such as POE sorbitan monooleate, POE sorbitan monostearate, and POE sorbitan tetraoleate; POE sorbitol fatty acid esters, such as POE sorbitol monolaurate, POE sorbitol monooleate, POE sorbitol pentaoleate, and POE sorbitol monostearate; POE glycerin fatty acid esters, such as POE glycerin monostearate, POE glycerin monoisostearate, and POE glycerin triisostearate; POE fatty acid esters, such as POE monooleate, POE distearate, POE dioleate, and ethylene glycol distearate; POE alkyl ethers, such as POE lauryl ether, POE oleyl ether, POE stearyl ether, POE behenyl ether, POE 2-octyldodecyl ether, and POE cholestanol ether; POE alkyl phenyl ethers, such as POE octyl phenyl ether, POE nonyl phenyl ether, and POE dinonyl phenyl ether; Pluaronic types, such as Pluronic; POE/POP alkyl ethers, such as POE/POP cetyl ether, POE/POP 2-decyltetradecyl ether, POE/POP monobutyl ether, POE/POP hydrogenated lanolin, and POE/POP glycerin ether; tetra-POE/tetra-POP ethylenediamine condensation products, such as Tetronic; POE castor oil hydrogenated castor oil derivatives, such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester, and POE hydrogenated castor oil maleic acid; POE beeswax/lanolin derivatives, such as POE sorbitol beeswax; alkanolamides, such as coconut oil fatty acid diethanolamide, lauric acid monoethanolamide, and fatty acid isopropanol amide; POE propylene glycol fatty acid esters; POE alkylamines; POE fatty acid amides; sucrose fatty acid esters; POE nonylphenyl formaldehyde condensation products; alkyl ethoxy dimethylamine oxides; and trioleyl phosphoric acid.

Other surfactants described above may be compounded in the range that does not cause any issues in stability and skin irritation. Examples thereof include anionic surfactants, such as fatty acid soaps, higher-alkyl sulfuric ester salts, POE triethanolamine lauryl sulfate, and alkyl ether sulfuric ester salts; cationic surfactants, such as alkyl trimethylammonium salts, alkyl pyridinium salts, alkyl quaternary ammonium salts, alkyl dimethylbenzyl ammonium salts, POE alkylamines, alkylamine salts, and polyamine fatty acid derivatives; and amphoteric surfactants, such as imidazoline amphoteric surfactants and betaine surfactants.

The moisturizer described above is not particularly limited. Examples thereof include xylitol, sorbitol, maltitol, chondroitin sulfate, hyaluronic acid, mucoitinsulfuric acid, caronic acid, atelocollagen, cholesteryl-12-hydroxystearate, sodium lactate, bile salts, dl-pyrrolidone carboxylate, short-chain soluble collagens, diglycerol (EO) PO adducts, Rosa roxburghii extract, yarrow extract, and melilot extract.

The higher alcohol described above is not particularly limited. Examples thereof include linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerol ether (batyl alcohol), 2-decyltetradecynol, lanolin alcohol, cholesterol, phytosterol, hexyldodecanol, isostearyl alcohol, and octyldodecanol.

The sequestrant is not particularly limited. Examples thereof include 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, and edetic acid.

The natural water-soluble polymer described above is not particularly limited. Examples thereof include plant-derived polymers, such as gum arabic, tragacanth gum, galactan, guar gum, carob gum, karaya gum, carrageenan, pectin, agar, quince seed (quince), algal colloid (algal extract), starch (rice, corn, potato, wheat), and glycyrrhizinic acid; microorganism-derived polymers, such as xanthan gum, dextran, succinoglucan, and pullulan; and animal-derived polymers, such as collagen, casein, albumin, and gelatin.

The semisynthetic water-soluble polymer is not particularly limited. Examples thereof include starch polymers such as carboxymethyl starch and methyl hydroxypropyl starch; cellulose polymers such as methyl cellulose, nitro cellulose, ethyl cellulose, methyl hydroxypropyl cellulose, hydroxyethyl cellulose, cellulose sodium sulfate, hydroxypropyl cellulose, sodium carboxymethylcellulose (CMC), crystalline cellulose, and cellulose powder; and alginate polymers such as sodium alginate and propylene glycol alginate.

The synthetic water-soluble polymer is not particularly limited. Examples thereof include vinyl polymers, such as polyvinyl alcohol, polyvinyl methyl ether, and polyvinyl pyrrolidone; polyoxyethylene polymers, such as polyethylene glycol 20000, polyethylene glycol 40000, and polyethylene glycol 60000; polyoxyethylene-polyoxypropylene copolymers; acrylic polymers, such as sodium polyacrylate, polyethylacrylate, and polyacrylamide; polyethyleneimine; and cationic polymers.

The inorganic water-soluble polymer is not particularly limited. Examples thereof include bentonite, AlMg silicate (Veegum), laponite, hectorite, and silicic anhydride.

The organic ultraviolet shielding agent is not particularly limited. Examples thereof include benzoic acid-based ultraviolet shielding agents, such as paraaminobenzoic acid (hereinafter, abbreviated as "PABA"), PABA monoglycerin ester, N,N-dipropoxy PABA ethyl ester, N,N-diethoxy PABA ethyl ester, N,N-dimethyl PABA ethyl ester, and N,N-dimethyl PABA butyl ester; anthranilic acid-based ultraviolet shielding agents, such as homomenthyl-N-acetyl anthranilate; salicylic acid-based ultraviolet shielding agents, such as amyl salicylate, menthyl salicylate, homomenthyl salicylate, octyl salicylate, phenyl salicylate, benzyl salicylate, and p-isopropanol phenyl salicylate; cinnamic acid-based ultraviolet shielding agents, such as ethylhexyl p-methoxycinnamate, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glycerylmono-2-ethylhexanoyl-diparamethoxy cinnamate; benzophenone-based ultraviolet shielding agents, such as 2,4-dihydroxybenzophenone, 2,2' -dihydroxy-4-methoxybenzophenone, 2,2' -dihydroxy-4,4' -dimethoxybenzophenone, 2,2' ,4,4' - tetrahydroxybenzophenone, 2-hydroxy-4-methoxybenzophenone, 2-hydroxy-4-methoxy-4' - methylbenzophenone, 2-hydroxy-4-methoxybenzophenone-5-sulfonate, 4-phenylbenzophenone, 2-ethylhexyl-4' -phenyl-benzophenone-2-carboxylate, 2-hydroxy-4-n-octoxybenzophenone, and 4-hydroxy-3-carboxybenzophenone; 3-(4'-methylbenzylidene)-d,l-camphor, 3-benzylidene-d,l-camphor, urocanic acid, urocanic acid ethyl ester, 2-phenyl-5-methylbenzoxazole, 2,2' -hydroxy-5-methylphenyl benzotriazole, 2-(2'-hydroxy-5'-t-octylphenyl)benzotriazole, 2-(2' -hydroxy-5' -methylphenyl)benzotriazole, dibenzalazine, dianisoylmethane, 4-methoxy-4'-t-butyldibenzoylmethane, and 5-(3,3-dimethyl-2-norbornylidene)-3-pentan-2-one.

Various kinds of extracts are not particularly limited. Examples thereof include Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of the various kinds of powders described above include bright coloring pigments, such as red oxide, yellow iron oxide, black iron oxide, mica titanium, iron oxide-coated mica titanium, and titanium oxide-coated glass flakes, inorganic powders, such as those of mica, talc, kaolin, sericite, titanium dioxide, and silica, and organic powders, such as polyethylene powder, nylon powder, crosslinked polystyrene, cellulose powder, and silicone powder. Preferably, for enhancing sensory characteristics and makeup retainability, a part or all of the powder component may be subjected to a water repellent treatment by a publicly known method using a substance such as a silicone, a fluorine compound, a metallic soap, an oily agent, or an acyl glutamic acid salt, for use.

Other chemical components are not particularly limited, and examples thereof include vitamins, such as vitamin A oil, retinol, retinol palmitate, inositol, pyridoxine hydrochloride, benzyl nicotinate, nicotinamide, DL-α-tocopherol nicotinate, magnesium ascorbyl phosphate, 2-O-α-D-glucopyranosyl-L-ascorbic acid, vitamin D2 (ergocalciferol), DL-α-tocopherol, dL-α-tocopherol acetate, pantothenic acid, and biotin; hormones, such as estradiol and ethynyl estradiol; amino acids, such as arginine, aspartic acid, cystine, cysteine, methionine, serine, leucine, and tryptophan; anti-inflammatory agents, such as allantoin and azulene; whitening agents, such as arbutin; astringents, such as tannic acid; refrigerants, such as L-menthol and camphor, sulfur, lysozyme chloride, and pyridoxine chloride.

A method for producing the cosmetic of the present invention is not particularly limited, and the cosmetic may be produced by a production method according to the form of the cosmetic. Specifically, for example, the cosmetic can be obtained by mixing the dispersion of the present invention with other components.

### Examples

The present invention will be specifically described hereinafter with reference to examples, but the present invention is not limited to these examples. In the following description, "%" and "part(s)" refer to "mass%" and "part(s) by mass", respectively, unless otherwise specified.

### Production Example of Primary Product Zinc Oxide Cake

80 g of FINEX-50 (available from Sakai Chemical Industry Co., Ltd.; primary particle size: 0.020 µm) was repulped in 1200 mL of a zinc acetate aqueous solution having a zinc acetate concentration of 0.135 mol/L, and thus a slurry was obtained. Subsequently, the temperature of the slurry was increased to 70°C over 42 minutes while the slurry was agitated, and then the slurry was aged at 70°C for 2 hours while being agitated. After the aging, filtration and water washing were performed. Subsequently, the obtained solid material was repulped in 3 L of water to form a slurry, the temperature of the slurry was increased to 95°C while the slurry was agitated, and heating and washing was performed at 95°C for 60 minutes while the slurry was agitated. After the heating and washing, filtering and water washing were performed, and thus a zinc oxide cake was obtained.

### Example 1

The zinc oxide cake obtained in Production Example was weighed for 30 g of pure zinc oxide, and repulped into pure water so that a powder concentration was 100 g/L to obtain a zinc oxide slurry. 0.45 g of 30% caustic soda was added to pure water, the mixture was heated at 90°C, and then 0.9 g (3.0% with respect to zinc oxide) of stearic acid (stearic acid available from FUJIFILM Wako Pure Chemical Corporation, plant-derived) was added to obtain an aqueous sodium stearate solution. The aqueous sodium stearate solution (90°C) was added to the zinc oxide slurry heated to 90°C, and the mixture was aged for 2 hours while maintaining the temperature at 90°C. After aging, the mixture was cooled to 70°C, adjusted to pH 7.0 with sulfuric acid, then filtered, washed with water, and dried at 120°C for 16 hours to obtain stearic acid-treated zinc oxide particles. The evaluation results of physical properties of the obtained particles and physical properties of the coating film were shown in Table 1.

The stearic acid has a melting point of 70°C.

### Example 2

Stearic acid-treated zinc oxide particles were obtained in the same manner as in Example 1 except that 0.3 g of 30% caustic soda was added to pure water, the mixture was heated at 90°C, and then 0.6 g (2.0% with respect to zinc oxide) of stearic acid was added to obtain an aqueous sodium stearate solution, and the mixture was filtered, washed with water, and then dried at 80°C.

### Example 3

Isostearic acid-treated zinc oxide particles were obtained in the same manner as in Example 1 except that stearic acid was changed to isostearic acid (isostearic acid (isomer mixture) available from FUJIFILM Wako Pure Chemical Corporation).

The isostearic acid has a melting point of 7°C.

### Example 4

Palmitic acid-treated zinc oxide particles were obtained in the same manner as in Example 1 except that stearic acid was changed to palmitic acid (palmitic acid available from FUJIFILM Wako Pure Chemical Corporation).

The palmitic acid has a melting point of 64°C.

### Comparative Example 1

Stearic acid-treated zinc oxide particles were obtained in the same manner as in Example 1 except that drying was performed at 80°C for 16 hours.

### Comparative Example 2

Stearic acid-treated zinc oxide particles were obtained in the same manner as in Example 1 except that the amount of 30% caustic soda was changed to 1.5 g, and the amount of stearic acid was changed to 3.0 g (10.0% with respect to zinc oxide).

### Comparative Example 3

The zinc oxide cake obtained in Production Example was dried at 120°C for 16 hours to obtain a zinc oxide powder. 30 g of the obtained zinc oxide powder was placed in a plastic bag, 1.2 g (4.0% with respect to zinc oxide) of stearic acid was added and mixed, the mixture was subjected to heat treatment at 120°C for 16 hours by using a dryer, and thus stearic acid-treated zinc oxide particles were obtained.

### Comparative Example 4

Stearic acid-treated zinc oxide particles were obtained in the same manner as in Example 1 except that the zinc oxide cake obtained in Production Example was changed to FINEX-30 (available from SAKAI CHEMICAL INDUSTRY CO., LTD., average particle size: 35 nm), the amount of 30% caustic soda was changed to 0.6 g, and the amount of stearic acid was changed to 1.2 g (4.0% with respect to zinc oxide).

### Comparative Example 5

Stearic acid-treated zinc oxide particles were obtained in the same manner as in Example 1 except that 0.45 g of 30% caustic soda was added to a zinc oxide slurry, the mixture was heated at 40°C, and then 0.9 g (3.0% with respect to zinc oxide) of stearic acid was added, and the mixture was aged for 2 hours while maintaining the temperature at 40°C.

### Comparative Example 6

Isostearic acid-treated zinc oxide particles were obtained in the same manner as in Comparative Example 2 except that stearic acid was changed to isostearic acid.

### Evaluation Method 1: Composition of Obtained Particles

The composition of the obtained particles in Table 1 shows a result of analysis by an X-ray diffractometer UltimaIII (available from Rigaku Corporation) having an X-ray tube with copper.

### Evaluation Method 2: Primary Particle Size

The primary particle size in the present specification is calculated as an average value of particle sizes obtained by measuring particle sizes of 250 particles randomly selected in a field of view of from 20000 to 50000 times a photograph taken by a transmission electron microscope JEM-1200EX II (available from JEOL Ltd.).

### Evaluation Method 3: BET Specific Surface Area

The BET specific surface area (m²/g) in Table 1 is a value measured by Full Automatic BET Specific Surface Area Analyzer Macsorb Model HM-1200 (available from Mountech Co., Ltd.).

### Evaluation Method 4: Carbon Content

The carbon content in Table 1 is a value measured by a carbon content measuring device CARBON ANALYZER EMIA-110 (available from HORIBA, Ltd.).

### Evaluation Method 5: Mode Diameter

In the present specification, the mode diameter is a value measured using a laser diffraction/scattering particle size distribution analyzer LA-960S (available from HORIBA, Ltd.). 0.1 g of the zinc oxide particles of each of Examples and Comparative Examples were added in 50 ml of isopropyl alcohol. A slurry obtained by dispersing the slurry by ultrasonic irradiation for 1 minute by using an ultrasonic homogenizer US-600E (available from Nihonseiki Kaisha Ltd.) was measured. The measurement based on volume was performed with the refractive index of the zinc oxide of each of Examples and Comparative Examples being 2.00 and the refractive index of isopropyl alcohol being 1.378.

When the mode diameter of the slurry subjected to ultrasonic dispersion for 1 minute is 0.30 µm or less, there are many particles in a state close to the primary particle size even in the case of weak dispersion; therefore, it means that the dispersibility is high, and when the mode diameter is more than 0.30 µm, it means that aggregation of particles is strong and the dispersibility is low.

### Evaluation Method 6: Water Repellency

80 mL of distilled water was charged in a 100 mL beaker, 0.2 g of the fatty acid-treated zinc oxide particles were floated and stirred 10 times by a spatula, turbidity of water was visually observed, and evaluation was performed based on the following criteria.
○: No turbidity was observed in the water, and the water was transparent
△: Slight turbidity was observed in the water, and the water was semi-transparent
×: White turbidity was observed in the water

### Evaluation Method 7: Apparent Density

The measurement was performed by a method described in JIS K 5101-12-1 "Test methods for pigments - Part 12: Apparent density or apparent specific volume - Section 1: Loose packing method".

### Evaluation Method 8: FT-IR

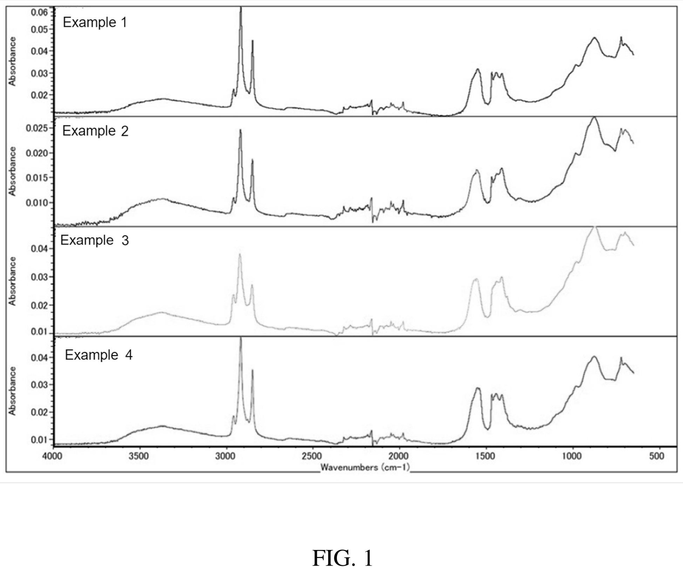

The FT-IR spectra in FIGS. 1 and 2 are spectra measured under the following conditions using a Fourier transform infrared spectrophotometer FT-IR Nicolet iS 10 (available from Thermo Fisher Scientific Inc.). The measurement was performed with a detector of DTGS KBr, a resolution of 4 cm⁻¹, and the number of times of scanning of 16 times.

An accessory device is a single reflection horizontal ATR Smart iTR, a use plate (IRE) is diamond crystal, and an angle of incidence is 45°.

A value of the absorbance I₁ is the absorbance of the maximum peak among CH₃ symmetric bending vibration, COO⁻symmetric stretching vibration, and CH₃ asymmetric bending vibration detected at from 1350 to 1500 cm⁻¹, and a value of the absorbance I₂ is a value of the absorbance of the maximum peak of COO⁻antisymmetric stretching vibration detected at from 1500 to 1600 cm⁻¹. When the value of (I₂/I₁) is from 0.8 to 1.2, it means that the fatty acid is bound to a zinc oxide surface, when the value is more than 1.2, it means that free fatty acid zinc is precipitated, and when the value is less than 0.8, it means that the fatty acid is not sufficiently bound to the zinc oxide surface.

In addition, a value of the absorbance I₃ is the absorbance of the peak of C = O stretching vibration detected at from 1700 to 1720 cm⁻¹, and a value of the absorbance I₄ is a value of the absorbance of the peak of C = O stretching vibration of a four-membered ring, a five-membered ring, or an acid anhydride detected at from 1750 to 1800 cm⁻¹. In this production process, no substance corresponding to a four-membered ring, a five-membered ring, and an acid anhydride is present, and therefore no peak is detected. Thus, when the value of I₃/I₄ is more than 1.2, it means that unreacted fatty acid remains.

### Examples 5 to 8 and Comparative Examples 7 to 12

The fatty acid-treated zinc oxide particle dispersion having the composition shown in Table 2 was prepared by the following production method, and for each sample, "initial viscosity", "temporal viscosity at 40°C", and "temporal stability" were evaluated, and the results are also shown in Table 2.

### Production Method

In a mayonnaise bottle, the fatty acid-treated zinc oxide particles of Examples and Comparative Examples, polyhydroxystearic acid (SALACOS HS-6C,available from The Nisshin OilliO Group, Ltd.), and isotridecyl isononanoate (SALACOS 913, available from The Nisshin OilliO Group, Ltd.) at a ratio shown in Table 2, and zirconia beads (0.5 mm, available from TORAY INDUSTRIES, INC.) in an amount of twice the total amount of components were put, and the mixture was well mixed, then fixed to a paint conditioner (available from Red Devil Inc.), and vibrated for 60 minutes to prepare a dispersion. The evaluation results of the physical properties of the obtained dispersion are shown in Table 2.

### Initial Viscosity

After dispersion, the dispersion was put in a 9 ml screw bottle, using a rotor No. 4 with a B-type viscometer (available from TOKYO KEIKI), and the viscosity (25°C) after 60 seconds from the start of rotation at 12 rpm was measured.

### Temporal Viscosity at 40°C

The dispersion was put in a 9 ml screw bottle and stored in a thermostatic bath at 40°C for 7 days. Using a rotor No. 4 with a B-type viscometer (available from TOKYO KEIKI), the viscosity after 60 seconds from the start of rotation at 12 rpm was measured.

### Temporal Stability

A state after storage in a 40°C thermostatic bath for 7 days was evaluated according to the following criteria.
⊚: No sedimentation and viscosity of less than 1000 mPa·s
○: No sedimentation and viscosity of 1000 mPa·s or more and less than 2000 mPa·s
△: No sedimentation and viscosity of 2000 mPa·s or more and less than 3000 mPa·s
×: There is sedimentation, or the viscosity is 3000 mPa·s or more

**[Table 1]**

| | | Example 1 | Example 2 | Example 3 | Example 4 | Comparative Example 1 | Comparative Example 2 | Comparative Example 3 | Comparative Example 4 | Comparative Example 5 | Comparative Example 6 |
|---|---|---|---|---|---|---|---|---|---|---|---|
| Surface treatment conditions | Presence or absence of primary product zinc oxide repulp | Presence | Presence | Presence | Presence | Presence | Presence | Absence | Presence | Presence | Presence |
| | Surface treatment agent | Stearic acid | Stearic acid | Isostearic acid | Palmitic acid | Stearic acid | Stearic acid | Stearic acid | Stearic acid | Stearic acid | Isostearic acid |
| | Amount (%) of surface treatment agent added to zinc oxide | 3.0 | 2.0 | 3.0 | 3.0 | 3.0 | 10.0 | 4.0 | 4.0 | 3.0 | 10.0 |
| | Fatty acid soda addition temperature | 90°C | 90°C | 90°C | 90°C | 90°C | 90°C | - | 90°C | 40°C | 90°C |
| Physical properties of p articles | Composition of obtained particles | Zinc oxide | Zinc oxide | Zinc oxide | Zinc oxide | Zinc oxide | Zinc oxide | Zinc oxide | Zinc oxide | Zinc oxide | Zinc oxide |
| | Primary particle size (µm) | 0.049 | 0.046 | 0.051 | 0.050 | 0.048 | 0.055 | 0.052 | 0.035 | 0.048 | 0.058 |
| | BET specific surface area (m²/g) | 16.1 | 16.8 | 16.4 | 16.7 | 16.2 | 11.6 | 16.3 | 19.8 | 17.3 | 7.92 |
| | Carbon content (%) | 2.30 | 1.48 | 2.21 | 2.28 | 2.30 | 7.35 | 2.97 | 3.01 | 2.34 | 6.71 |
| | Mode diameter (µm) | 0.14 | 0.14 | 0.14 | 0.14 | 8.20 | 7.18 | 0.11 | 5.47 | 6.28 | 0.32 |
| | Water repellency | ○ | ○ | ○ | ○ | ○ | ○ | ○ | ○ | ○ | ○ |
| | Apparent density (g/ml) | 0.96 | 0.98 | 0.82 | 0.94 | 0.99 | 0.84 | 1.08 | 0.71 | 0.95 | 0.61 |
| IR peak | Absorbance I ₁ | 0.030 | 0.017 | 0.030 | 0.026 | 0.029 | 0.065 | 0.033 | 0.029 | 0.031 | 0.132 |
| | Absorbance I ₂ | 0.032 | 0.017 | 0.029 | 0.029 | 0.041 | 0.087 | 0.037 | 0.031 | 0.032 | 0.172 |
| | I₂/I₁ | 1.07 | 1.00 | 0.97 | 1.10 | 1.41 | 1.34 | 1.12 | 1.07 | 1.03 | 1.30 |
| | Absorbance I ₃ | 0.0105 | 0.0080 | 0.0115 | 0.0090 | 0.0105 | 0.0240 | 0.0160 | 0.0095 | 0.0110 | 0.0550 |
| | Absorbance I ₄ | 0.0110 | 0.0075 | 0.0105 | 0.0090 | 0.0095 | 0.0250 | 0.0080 | 0.0092 | 0.0110 | 0.0520 |
| | I₃/I₄ | 0.95 | 1.07 | 1.10 | 1.00 | 1.11 | 0.96 | 2.00 | 1.03 | 1.00 | 1.06 |

**[Table 2]**

| Component | | Example 5 | Example 6 | Example 7 | Example 8 | Comparative Example 7 | Comparative Example 8 | Comparative Example 9 | Comparative Example 10 | Comparative Example 11 | Comparative Example 12 |
|---|---|---|---|---|---|---|---|---|---|---|---|
| Fatty acid treatment of Example 1 Zinc oxide particles | | 65% | - | - | - | - | - | - | - | - | - |
| Fatty acid treatment of Example 2 Zinc oxide particles | | - | 65% | - | - | - | - | - | - | - | - |
| Fatty acid treatment of Example 3 Zinc oxide particles | | - | - | 65% | - | - | - | - | - | - | - |
| Fatty acid treatment of Example 4 Zinc oxide particles | | - | - | - | 65% | - | - | - | - | - | - |
| Fatty acid treatment of Comparative Example 1 Zinc oxide particles | | - | - | - | - | 65% | - | - | - | - | - |
| Fatty acid treatment of Comparative Example 2 Zinc oxide particles | | - | - | - | - | - | 65% | - | - | - | - |
| Fatty acid treatment of Comparative Example 3 Zinc oxide particles | | - | - | - | - | - | - | 65% | - | - | - |
| Fatty acid treatment of Comparative Example 4 Zinc oxide particles | | - | - | - | - | - | - | - | 65% | - | - |
| Fatty acid treatment of Comparative Example 5 Zinc oxide particles | | - | - | - | - | - | - | - | - | 65% | - |
| Fatty acid treatment of Comparative Example 6 Zinc oxide particles | | - | - | - | - | - | - | - | - | - | 65% |
| Polyhydroxystearic acid | | 3% | 3% | 3% | 3% | 3% | 3% | 3% | 3% | 3% | 3% |
| Isotridecyl isononanoate | | 32% | 32% | 32% | 32% | 32% | 32% | 32% | 32% | 32% | 32% |
| Physical properties of dispersion | Initial viscosity (mPa·s) 12 rpm | 100↓ | 100↓ | 500 | 100↓ | 750 | High viscosity Unseparable | High viscosity Unseparable | 1960 | 880 | High viscosity Unseparable |
| | Viscosity (mPa·s) after 7 days at 40°C 12 rpm | 1130 | 500 | 100↓ | 1860 | 2610 | - | - | 11320 | 6240 | - |
| | Temporal stability | ○ | ⊚ | ⊚ | ○ | Δ | - | - | × | × | - |

Table 1 indicates that the fatty acid-treated zinc oxide particles of Examples are excellent in dispersibility and have sufficient water repellency. In addition, Table 2 indicates that no remarkable thickening was observed in both the initial viscosity and the temporal viscosity, and the stability was also excellent.

### Example 9 Sunscreen O/W cream

Using the components shown in the following Table 3, a sunscreen O/W cream was obtained by the following production method.

### Production Method

(1) Uniformly mixing the components 1 to 6, and heat the mixture to dissolve.
(2) Uniformly mixing the components 7 to 12 and heating the mixture.
(3) Adding the aforementioned (1) to (2), and then emulsifying and cooling the mixture to obtain a sunscreen O/W cream.

**[Table 3]**

| | Component | (%) |
|---|---|---|
| 1 | Fatty acid-treated zinc oxide oil dispersion (Example 5) | 23.1 |
| 2 | Hydrogenated polyisobutene | 9.9 |
| 3 | Dimethicone | 1.5 |
| 4 | Behenyl alcohol | 0.7 |
| 5 | Sorbitan sesquioleate | 0.5 |
| 6 | Glyceryl stearate | 0.3 |
| 7 | (Na acrylate/Na acryloyldimethyltaurate) copolymer (Note 1) | 0.6 |
| 8 | Polysorbate 60 | 1.2 |
| 9 | Bentonite | 0.1 |
| 10 | Plant-derived cellulose (2%aq) (Note 2) | 2.5 |
| 11 | 1,3-Butylene glycol | 6.5 |
| 12 | Purified water | 53.1 |

| | | |
|---|---|---|
| (Note 1) (Na acrylate/Na acryloyldimethyltaurine) copolymer: SIMULGEL EG QD (available from SEPPIC) (Note 2) Plant-derived cellulose (2%aq): RHEOCRYSTA C-2SP (available from DKS Co. Ltd.) | | |

The sunscreen O/W cream of Example 9 obtained as described above was an organic UV absorber-free sunscreen O/W cream that is stable without any changes over time, has a smooth sensory feel with good spread over the skin when applied, has no stickiness or "squeaky" feel on the skin, has high transparency, and has excellent persistence of the sunscreening effect.

### Industrial Applicability

The dispersion of the present invention can be suitably used by being blended in a cosmetic.

## Claims

1. A fatty acid-treated zinc oxide particle, having, in an FT-IR spectrum measured using a total reflection measurement method (ATR method), a ratio (I₂/I₁) of a maximum absorbance (I₂) at from 1500 to 1600 cm⁻¹ to a maximum absorbance (I₁) at from 1350 to 1500 cm⁻¹ of from 0.8 to 1.2, and a ratio (I₃/I₄) of a maximum absorbance (I₃) at from 1700 to 1720 cm⁻¹ to a maximum absorbance (I₄) at from 1750 to 1800 cm⁻¹ of 1.2 or less,
wherein a primary particle size measured with a transmission electron microscope is 0.1 µm or less, and
a volume-based mode diameter, according to a laser diffraction scattering method, of a dispersion liquid obtained by adding the powder to isopropyl alcohol and dispersed by ultrasonic irradiation using an ultrasonic homogenizer with a rated output of 600 W and a vibration amplitude of 100% at normal temperature for 1 minute is 0.30 µm or less.

2. The fatty acid-treated zinc oxide particle according to claim 1, wherein the fatty acid is at least one selected from the group consisting of stearic acid, isostearic acid, and palmitic acid.

3. A method for producing a fatty acid-treated zinc oxide particle, comprising:
Step (1) preparing a slurry by repulping zinc oxide particles as a primary product in a dispersion medium;
Step (2) neutralizing a fatty acid with a base to obtain a fatty acid salt aqueous solution; and
Step (3) performing a surface treatment by adding the obtained fatty acid salt aqueous solution to the zinc oxide slurry,
wherein the zinc oxide particles as the primary product are obtained by a production method including aging fine zinc oxide particles in water, in which a zinc salt is dissolved, and are subjected to Step (1) without drying, and Steps (2) and (3) are performed at a temperature equal to or higher than a melting point of the fatty acid.

4. The method for producing a fatty acid-treated zinc oxide particle according to claim 3, wherein in the zinc oxide particle as the primary product, a median diameter, according to a laser diffraction scattering method, of a dispersion liquid obtained by adding the powder to a 0.0025 mass% sodium hexametaphosphate aqueous solution and dispersed by ultrasonic irradiation using an ultrasonic homogenizer with a rated output of 600 W and a vibration amplitude of 100% at normal temperature for 3 minutes is 0.30 µm or less.

5. A dispersion, comprising the fatty acid-treated zinc oxide particles according to claim 1 or 2.

6. A cosmetic, comprising the fatty acid-treated zinc oxide particles according to claim 1 or 2.
